# EUROPEAN PATENT APPLICATION

(11) **EP 0 577 362 A2**
(43) Date of publication of application: **05.01.1994**
(21) Application number: 93305020.5
(22) Date of filing: 28.06.1993
(51) Int. Cl.: G06F 13/372, G06F 3/06

(54) **An expanded architecture for image storage and distribution**

(30) Priority: 01.07.1992 US 907303
(71) Applicant: LORAL AEROSPACE CORPORATION, New York, NY 10016 (US)
(72) Inventor: Wilson, Dennis L., Palo Alto, CA 94301 (US); McReynolds, John M., San Jose, CA 95122 (US); Glicksman, Robert A., San Jose, CA 95136 (US); Carlson, Richard A., Los Altos, CA 94924 (US)
(74) Representative: Vaufrouard, John Charles

(57) **Abstract**

An architecture (20) that provides for the connection of storage media and disks to multiple workstations (15) or ports, and which overcomes rate incompatibilities of the various components in the architecture (20). The rate incompatibilities are overcome such that one storage unit (13) may be connected to many workstations (15) so that individual workstations (15) operate as though they were the only connection to the storage unit (13). The storage unit (13) is operated in parallel using a redundant array of storage disks to supply data at a very high rate. The disks are connected through a high speed backplane bus (24) to rate buffers on input and output cards. The input and output cards are connected by high speed optical data links (26) to interface cards in the workstations (15) that accept data transmitted at high data rates and deliver the data to the workstation memory. Each input or output card is time shared by a number of workstations (15). The data is moved through the input or output card on the storage unit (13) so quickly that there is a low probability of two workstations (15) wanting to move data through the same port at the same time.

## Description

### BACKGROUND

The present invention relates generally to image storage architectures, and more particularly, to an image storage and dissemination architecture that provides for collection, storage, and distribution of images and other large data blocks to a large number of users and that overcomes inherent rate incompatibilities among components of the architecture.

The issue of interconnecting multiple processors has been addressed in numerous patents. The patents cited below generally are not concerned with connecting systems that operate at different rates. They may incidentally connect systems with different rates, but their primary goal is the support of a multiported memory function. The peripherals that are connected to the system are located at a very short distance from the memory of the system, contrasted with the present invention wherein the systems and may be separated by several kilometers.

U.S. Patent No. 3,639,909 issued to Hauck, et al., entitled "Multichannel Input/Output Control with Automatic Channel Selection" is concerned with routing of data from memory to peripherals. The data rates of the peripherals is not an important consideration. The primary contribution is the routing of the data from the memory through multiple control units to a larger number of I/O devices. A tag is added to the data moving to the peripheral to permit the system to move the data through the control units and a switch to the peripheral.

U.S. Patent No. 3,761,879 issued to Brandsma, et al. entitled "Bus Transport System for Selection Information and Data" provides for an effective switch between memory and processors. The switch allows several processors to access several storage modules. The present invention is much more than a switch. It balances the data rates of the units, taking into consideration the fact that computer workstations require data only occasionally.

U.S. Patent No. 4,654,788 issued to Boudreau, et al. entitled "Asynchronous Multiport Parallel Access Memory System for use in a Single Board Computer System", provides for a multiported memory with priority access. The scheme uses multiple system buses to increases the effective bandwidth from the memory to the I/O devices. A basic assumption is that the I/O devices are much slower than the memory. In contrast, the present invention connects a storage unit to workstations over a distance such that the data rates in the workstations are very comparable the data rates from disks of the storage unit. Maintaining the high speed transfer through to the workstation is a central concept of the present invention. The present invention moves large blocks of data in a timely way such that each of the many workstations can consider that it is the only one using the storage unit.

U.S. Patent No. 4,484,262 issued to Sullivan, et al. entitled "Shared Memory Computer Method and Apparatus" is concerned with distributing data over a number of storage module with a switch between the storage modules and the sources such that the bandwidth of the storage modules is effectively used. This concept uses a hashing scheme to spread the data over the storage modules, thus making sure that no one memory module is overloaded. The data is moved in small blocks. In contrast, the present invention permits access by many workstations to one very large storage module. There is no hashing involved, since there is only one storage module. The present invention moves large blocks of data in contrast to the small blocks of data moved by the Sullivan, et al. system.

U.S. Patent No. 4,707,781 issued to Sullivan, et al. entitled "Shared Memory Computer Method and Apparatus" provides for a switching structure to be used with the device of the U.S. Patent No. 4,484,262 cited above. The switching structure permits any one source to be connected to any memory module. The switch is a straightforward space switch such as is used in the communication industry on a regular basis. In contrast, the present invention uses time division multiplexing of data over a backplane.

U.S. Patent No. 4,780,812 issued to Freestone, et al. entitled "Common Memory System for a Plurality of Computers" provides a mechanism for moving one "word" at a time from a memory to each of several processors on a priority basis. In contrast, the present invention moves large blocks between a disk storage module and workstation memory. The connection can cover a long distance with all of the latencies involved in the long distance. If the scheme of the Freestone patent were used in the present invention the memory rates would slow down by an order of magnitude, losing the memory bandwidth in the long distances involved.

Thus it is an objective of the present invention to provide for an image storage and dissemination architecture that provides for the collection, storage, and distribution of images and other large data blocks to a large number of users and that overcomes inherent rate incompatibilities between components of the architecture.

### SUMMARY OF THE INVENTION

The structure of the architecture of the present invention provides for the connection of storage media and disks to multiple workstations or ports. The difficulties overcome by the present invention are rate incompatibilities of the various components in the architecture. The rate incompatibilities are overcome in such a way that one storage unit can be connected to many workstations in such a way that individual workstations operate as though they were the only connection to the storage unit. The storage unit is operated in parallel using a redundant array of inexpensive disks to supply data at a very high rate. The disks are connected through a high speed backplane bus to rate buffers on input and output cards. The input and output cards are connected by high speed data links to interface cards in the workstations that accept data transmitted at high data rates and deliver the data to the workstation memory. Each input or output card is time shared by a number of workstations. The data is moved through the input or output card on the storage unit so quickly that there is a low probability of two workstations wanting to move data through the same port at the same time.

In a preferred embodiment of the present invention, the data rates are balanced to achieve the highest system performance possible. The data rate in the workstation backplane establishes the basic desired operating rate. The buffer card in a storage unit, the interface card in the workstation, and the connection path therebetween, operate at about 60% of this backplane rate. The backplane rate of the storage unit is at least three times the port rate. The aggregate disk rate is approximately twice the backplane rate.

The use of compression in the interface cards of the workstations makes the rate balance easier to achieve. A simple compression scheme achieves a compression ratio of 2.5 to 3.0 and operates at a high rates. The compression lets the storage unit, the communication link to the workstation, and the interface card in the workstation operate 2.5 to 3.0 times slower than described above.

In an implementation that is adapted for use in a medical imaging application, the backplane rate of the workstations is 30 megabytes per second. The port rate on the storage unit with the connection to the workstation interface card operates at 12.5 megabytes per second. Overhead on the interface card reduces the port rate to about 10 megabytes per second. The data rate after the compression has been removed is about 25 megabytes per second, comfortably below the capacity of the backplane so there is no need for a large buffer on the interface card.

The data rate on the backplane of the working storage unit is 40 megabytes per second, four times the effective data rate of the ports of 10 megabytes per second. The aggregate data rate of the disks is 60 megabytes per second of data, 1.5 times as fast as the backplane.

The prior art patents cited in the Background are generally concerned with the closely coupled connection of multiple memory modules to multiple processors. The basic assumption in all of these patents is that the processor or I/O port is close to the memory so that the delays in moving data to the processor or I/O port from the memory are small. When the memory has a large overhead, such a disk, most of the approaches of the prior art patents are not effective. The data transferred in the present invention must be moved in large blocks, not small blocks, or the overhead of the seek operations and disk latency will be overwhelming.

When the destination (workstation) is distant, the approaches of the prior art patents are not effective. A backplane bus has a bandwidth that is comparable to the reciprocal of the bus length. To connect workstations directly to a backplane over distances of kilometers reduces the bandwidth to a few megabits per second from the data rates of more than 600 megabits per second achieved in the present invention. The present invention achieves average data transfer rates to the workstations of more than 100 megabits per second with very low contention for the storage unit.

Several workstations may share one port of the storage unit. To move a 5 megabyte block of data requires less than one half second. Two to twenty workstations may be connected to one port depending on how often each workstation needs 5 megabyte blocks of data. None of the above-cited patents has provision for sharing a port on the storage unit to achieve connections to hundreds of workstations.

### BRIEF DESCRIPTION OF THE DRAWINGS

The various features and advantages of the present invention may be more readily understood with reference to the following detailed description taken in conjunction with the accompanying drawings, wherein like reference numerals designate like structural elements, and in which:
Fig. 1 shows a conventional digital data collection, storage, and dissemination system architecture;
Fig. 2 shows a detailed illustration of a digital data collection, storage, and dissemination system architecture in accordance with the principles of the present invention that is an improvement to the architecture of Fig. 1; and
Fig. 3 shows the details of the storage unit of the system of Fig. 2.

### DETAILED DESCRIPTION

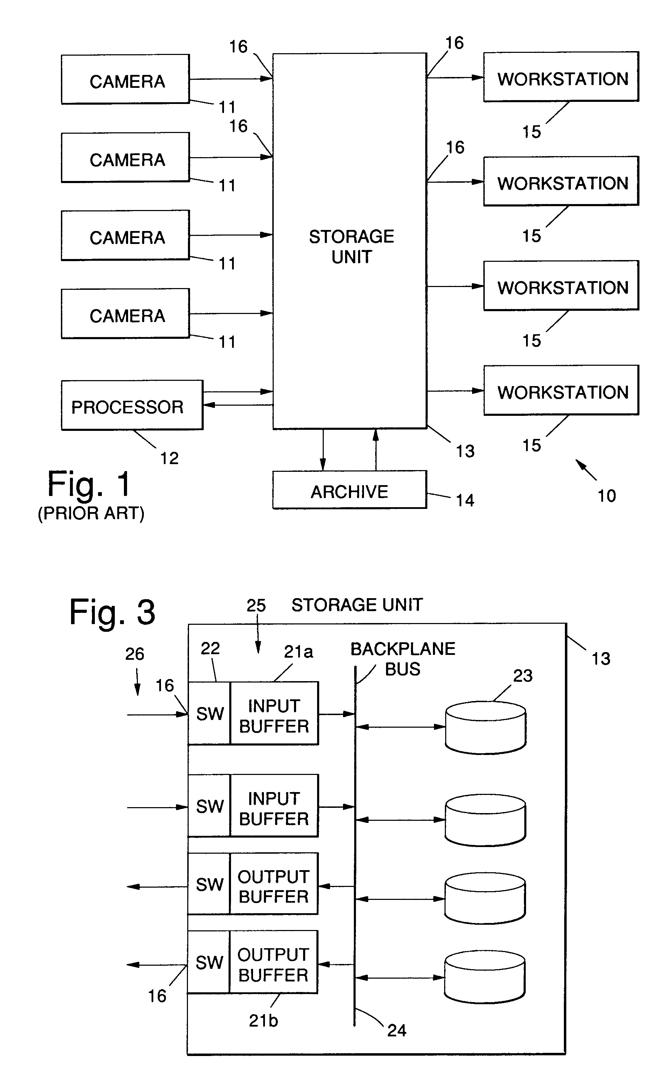

Referring to the drawing figures, Fig. 1 shows a conventional digital data collection, storage, and dissemination system 10. This conventional system 10 moves data from image sources 11 to a storage unit 13, to workstations 15, to a processor 12, or to an archive 14. Images are formed by an image source 11, such as a camera 11. The camera 11 may be a TV camera, an infrared imager, an optical imager, an Xray machine, a synthetic aperture radar, or other image source. The images are digitized and moved through the system 10 as a block of digital data. Typically images are very large blocks of data. A small image is a TV image that is 512x512 pixels or 262,144 pixels. In other applications, the images are considerably larger. For example, a typical Xray image is 2000 x 2500 pixels. The pixels may be quantized using a few bits or many bits per pixel. Typically 8 bits are used in many applications. In other applications there may be 12 bits per pixel.

The system 10 moves the pixels in digital form from the source 11 to the storage unit 13 where the images are held until they are to be viewed. When an operator is ready, a request is made for an image or set of images to be transferred to the operator's workstation 15 for viewing. The operator may also call up the storage unit 13 from which they must be retrieved. If a desire to view the historical images is known ahead of time, the images may be moved from the archive 14 to the storage unit 13 before the operator requests the images. Otherwise, the historical images must be retrieved from the archive 14 on request from the operator.

Other processing elements may be used in the system 10. These elements may include processors that compress the images prior to storage in the archive 14, expand the compressed images on retrieval from the archive 14, filter the images, enhance the images, register one image to another, and other processing functions.

In a typical installation the storage unit 13 has input/output ports 16 that connect to each of the cameras 11, to the processor 12, to the archive 14, and to each of the workstations 15. The result is a very large number of ports 16 into the storage unit 13. As long as there are only a few cameras 11, a few processors 12, and a few workstations 15, there can be one or two of these components connected to any one input/output port 16. As the number of components in the system 10 increases to over twenty, for example, the number of input/output ports 16 on the storage unit 13 increases until the system 10 is uneconomic.

Viewing the problem from a different perspective, the camera 11 must form an image and transfer the image to the storage unit 13. The operator retrieves the image at a workstation, then spends some time viewing the image. Similarly, the archive 14 spends some time locating the image, then transfering the image to the storage unit 13. The processors 12 retrieve the image, then spend some time processing the image.

Using a workstation 15 for illustration, an operator typically wants to retrieve an image in a short time, say a few seconds, for example, or the operator will become upset because of the relatively long perceived waiting time to retrieve the image. The operator then looks at the image, drawing conclusions and taking actions based on the information contained in the image. If, or example, the image is retrieved in one second and the operator takes 30 seconds to look at the image, the input/output port to the storage unit 13 is used only 3.3% of the time spent in working with the image.

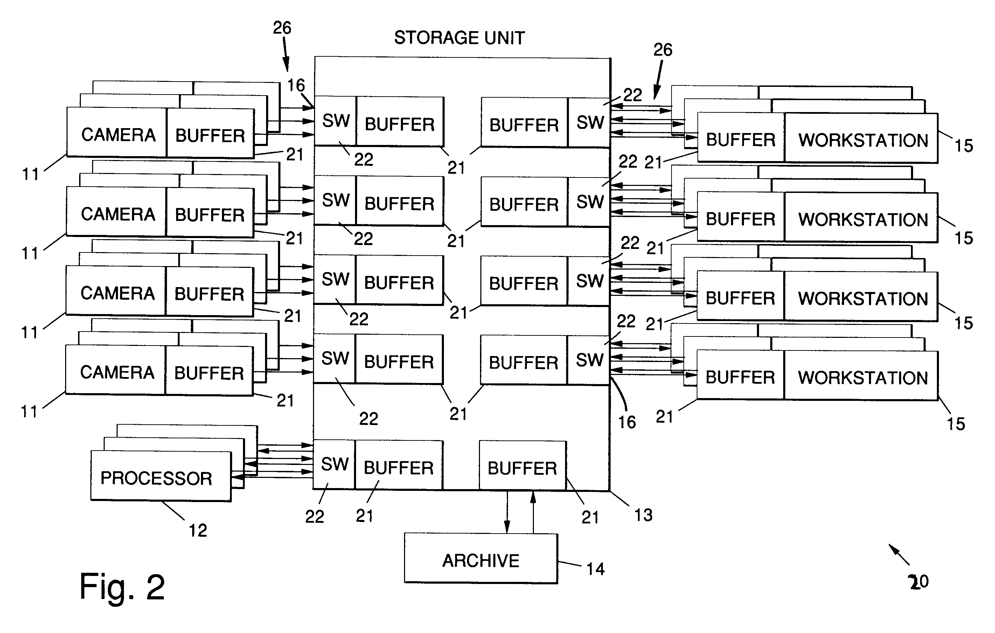

With reference to Fig. 2, the present architecture 20 or system 20 expands the number of elements that can be serviced in the conventional system 10 by an order of magnitude by adding switches 22, and adding buffers 21 to permit the rapid movement of data between the components of the system 20. In practice this means that one storage unit 13 supports tens to hundreds of workstations 15. There may also be many (>20, for example) cameras 11 in the system 11.

The present storage and distribution architecture 20 is shown in detail in Fig. 2. The present architecture 20 expands the number of devices (cameras 11, processors 12, and workstations 15, and hereinafter referred to as devices 11, 12, 15) that can be serviced by time sharing the input/output ports 16 on the storage unit 13. If each of the input/output ports 16 is used only a small percentage of the time by one of the devices 11, 12, 15 that are connected to the input/output port 16, several respective devices 11, 12, 15 may be connected to a particular input/output port 16 through the switch 22. As long as the utilization of the input/output port 16 to support the total load on the input/output port 16 is kept low, each of the devices connected to the input/output port 16 will operate essentially as though it were the only unit connected to the input/output port 16.

Two types of elements are added to the conventional system 10 to arrive at the present system 20, namely the switch 22 and a high-speed buffer 21. The buffers 21 may be made as integral parts of the storage unit 13, cameras 11, processors 12, workstations 15, and archive 14 connected to the storage unit 13. The switch 22 is added to each port of the storage unit 13

The central element of the storage unit 13 is a storage device 23, typically comprising a hard disk, for example. The storage unit 13 has an architecture shown in Fig. 3. The storage unit 13 comprises a high speed backplane 24 to distribute data to each of the storage devices 23. The backplane 24 is coupled between the buffers 21 and the storage devices 23.

The storage device 23 accepts data through an input interface 25 comprising a switch 22 and an input buffer 21a. The data in the input buffer 21a is organized into a wide word with error correction and each bit of the word is written to one of the storage devices 23. The storage unit 13 may use a 32 bit wide backplane 24 with an additional 7 bits of error correction. The data rate for each bit is 10 MHz. The total data rate is 320 Mbits per second.

The disk comprising the storage device 23 has associated track buffers that may be filled at rates ranging from 10 to 25 Mbits per second depending on the kind of disk. The disks also have an overhead including track seek and rotational latency that must be overcome before the data can be moved at the high data transfer rates.

The overhead imposed by the storage device 23 requires a preferred format structure for the data storage and retrieval provided by the storage device 23. Once the data starts moving, the storage device 23 is more efficient if a large block of data is moved at one time. If a small block of data is moved, each disk of the storage device 23 spends more time seeking the track and waiting for rotational latency than it does in transferring data. For the storage device 23, a nominal block size of about 1 MBytes is the size at which the overhead is equal to the data transfer time. A 50% efficiency size depends on the disk model used, since faster disks have a larger block size for 50% efficiency. In what follows below, 2.5 MBytes of data shall be the block size that is used. The storage device 23 is about 70% efficient with one hard disk with this block size. It takes 62.5 milliseconds to transfer the data from disk track buffers over the backplane 24 once a data transfer cycle has started. The data transfer cycle from the start of a seek to the arrival of data in an output buffer 21b is about 90 milliseconds. The average data transfer rate that is supported over the backplane 24 is 28 MBytes per second under these conditions.

To achieve a throughput rate of 28 MBytes per second, a request for service for the backplane 24 must be ready each time a data transfer cycle is completed. The only way that there can be a request for service waiting 100% of the time is for the individual devices 11, 12, 14 to have requests for service queued far into the future. The waiting time for a particular request is therefore very large.

An appropriate view of the storage device 23 is that the backplane 24 is a single server with a 2.5 MByte block serving time of 90 milliseconds, or 11.1 services per second. With this in mind, the storage device 23 is loaded to 30% to 50% of its capacity to ensure that the waiting time for a request in the queue is small. Thirty percent of the capacity of 28 MBytes per second, or an average rate is 8.4 MBytes per second, is a very large average rate for image data transfer.

The average rate required by typical cameras 11, workstations 15, processors 12, or archives 14 is quite low. A device 11, 12, 14, 15 that generates a 2k x 2k x 12 bit pixel picture once every 30 seconds has an average input rate of 0.2 MBytes per second. The rate corresponds to 0.08 service blocks per second compared with the rate of 11.1 service blocks per second that can be supplied. Such a device 11, 12, 14, 15 requires 0.72% of the capacity of the storage device 23.

Similarly, operators at the workstations 15 typically require a small percentage of the resources of the storage unit 13 on the average to view the images. An operator might look at 2 of the 2k x 2k x 12 bit images in one minute. At the end of this period of time looking at images, the operator will take some action based on the content of the images that might take as much time as the time taken to look at the images. As a consequence, the average rate at which the data is used is 6 MBytes per minute or 0.1 MBytes per second. The workstation 15 requires 0.36% of the services of the storage unit 13.

At these rates, in a typical application, the storage unit 13 supports forty workstations 15 and twenty cameras 11 before the utilization of the backplane 24 is above 30% of the capacity of the storage unit 13. To have sixty direct connections to the backplane 24 is not reasonable, and is not required using the switches 22 and buffers 21 of the present invention.

The switches 22 and buffers 21 will now be described in detail. If the input/output ports 16 on the storage unit 13 are time-shared across several devices 11, 12, 14, 15, the number of input/output ports 16 may be reduced. The input/output ports 16 themselves are relatively complex, requiring error correction, buffering and manipulation of the data. The switch 22 is very simple and permits the input/output ports 16 to be shared.

In order for the input/output port 16 to be shared effectively, the utilization of the input/output port 16 by any one device 11, 12, 14, 15 must be short and be should a small percentage of the capacity of the input/output port 16. For the storage unit 13, the highest data rate for a input/output port 16 is 100 Mbits per second, 31.25% of the data rate for the backplane 24. The time to move one 2.5 MByte block is 200 milliseconds through the output port 16 at this rate. The input/output port 16 capacity is 5 service blocks per second.

Some devices 11, 12, 14, 15 generate data or accept data at very low rates. For example, a device 11, 12, 14, 15 that uses an Ethernet connection is often restricted to a data transfer rate of several hundred KBytes per second. The connection of a device 11, 12, 14, 15 with this slow rate to an input/output port 24 uses up the port 16, but results in about 200 KBytes per second data transfer rate, far below the rate of 12.5 MBytes per second that the input/output port 16 is capable of supporting.

The addition of the input buffer 21a to a device 11, 12, 14, 15 permits data to be accumulated in the input buffer 21a at a slow rate until transfer to the storage unit 13 can be made at the high rate. The storage unit 13 can then switch between the buffers 21 attached to different devices 11, 12, 14, 15, achieving a data transfer rate that is much greater.

For an example calculation, a source rate (camera 11 transfer rate) is 0.08 service blocks per second and the workstation rate is 0.04 service blocks per second. One input/output port 16 moves 5 service blocks per second. As for the backplane 24, the utilization of the input/output port 16 is held to a small percentage of the capacity of the input/output port 16 in order that the time waiting for the input/output port 16 to be free be small. The rate of the input/output port 16 is only 30% of the rate of the backplane 24, so the delay in moving data is three times as long. It is appropriate to hold the utilization of the input/output port 16 to about 15% in order that the delays waiting for the port 16 not be overwhelming compared to the other delays in the system 20. With this restriction the average rate for a input/output port 16 may be as large as 0.75 service blocks per second.

The sources (cameras 11) are connected to input ports 16 (inut buffer 21a) and the workstations 15 are connected to output ports 16 (output buffer 21b). For sources such as the cameras 11, nine devices may be connected to a input/output port 16 before the average rate exceeds 0.75 service blocks per second. For the workstations 15, eighteen devices may be connected to a input/output port 16 before the average rate exceeds 0.75 service blocks per second.

The fact that devices 11, 12, 14, 15 must wait to use a selected input/output port 16 means that each device 11, 12, 14, 15 must be able to wait until the port 16 is free before transferring data. The buffer 21 in each device 11, 12, 14, 15 provides the capability to wait before the data transfer. Some devices 11, 12, 14, 15, such as streaming tape units, for example, cannot wait for the input/output port 16 without a buffer 21. Once the tape is moving, the data must be available to be recorded or the gaps will be created in the tape if recording; this reduces the overall capacity of the store on tape. On the other hand, if receiving data from tape and the buffer 21 is not sufficiently large, then data can be lost.

The waiting time for service will now be described. If the backplane 24 is used 30% of the time, 70% of the time the backplane 24 will be waiting for the next request for service. If the backplane 24 is in use, the probability of another request for service is about 30%, with a cumulative probability of 9%. In terms of waiting time, this means that 70% of the time there will be no wait for the backplane 24. Thirty percent of the time there will be a wait for at least one service to be completed, and about 9% of the time at least two service requests must be completed. The probability of having to wait for a longer time goes down exponentially. The average wait time is dominated by the first two terms, 0.7 * zero wait + 0.3 *one wait. The wait is 90 milliseconds if the service has just started. On the average, the service will be half over when a new request appears, so the average wait is about 0.3*45 milliseconds or 13.5 milliseconds, a very small wait for the backplane 24.

When the analysis is extended to the input/output port 16, the analysis is the same, but the wait times are longer and the probabilities lower. The upper limit is chosen at 15% loading of the input/output port 16. The time to service the input/output port 16 is 200 milliseconds. The average wait is roughly 0.85 * zero wait + 0.15 * one wait. As before the average wait if the input/output port 16 is busy is one half the service time of the input/output port 16 or 100 milliseconds. The average wait is 0.15 * 100 milliseconds or 15 milliseconds, comparable to the wait for the backplane 24. The total average wait is 13.5 + 15 milliseconds or 28.5 milliseconds, an acceptably short time.

The wait time is not zero. The scheme to switch devices to share a single port 16 requires that each of the devices 11, 12, 14, 15 is able to wait for a short time, up to several hundred milliseconds if necessary. The buffers 21 provide the capability to wait, when required, in addition to the high speed data transfers.

On the fly compression of images by a factor of two is comparatively easy to perform. Implementation of a modified Huffman coding scheme for images typically achieves 2.5 to 3.0 to one compression. If the data is compressed before it is stored in the storage unit 13, one service block holds twice as many pixels of the image. The number of service blocks required is thus reduced by a factor of two. The time to move an image is also decreased by a factor of two for the backplane 24 and for the input/output port 16. The size of the service block that has is used will move 3.3 Mpixels that are 12 bits deep with a compression of at least 2:1. A 5 Mpixel image is a large image and requires only two service blocks. Smaller 1000 x 1000 pixel images may be grouped into sets of three images for storage in the service block.

When the images are as small as TV images, 512 x 512 x 8 bit pixels or 262 KBytes uncompressed, 19 compressed images fit into one service block. 1.57 service blocks per second are required to support the TV image rate of 30 images per second for prolonged periods. A single input/output port 16 is used with this data rate, occupying 31% of the port maximum rate. However the rate of the storage unit 13 is 14.1% of its capacity and the storage unit 13 is able to support the output of the data at the same time that the data is input through several other input/output port s 16, as long as the total rate is less than about 50% of the 11.1 service blocks per second that the storage unit 13 supports.

The use of on-the-fly compression permits the expansion of the number of devices 11, 12, 14, 15 connected to the storage unit 13 to forty and eighty workstations 15 in a typical system 20. The number of devices serviced is expanded by a factor of two with compression.

The implementation of the buffers 21, the high speed connections, and the switches 22 may be done in a number of different ways. One approach is to connect the parallel interface of the storage unit 13 (comprising the plurality of input/output ports 16) to an electrical switch backplane 24. The output of each port 16 may be used to drive a separate parallel to serial converter circuit that feeds an optical fiber communication channel with a high speed serial stream. The individual fibers of a fiber optic cable 26 connect each of the distributed workstations 15 to the storage units 13. At the workstation 15, there is an optical receiver and a serial to parallel converter to change back to the parallel format. The use of the fiber optic cable 26 permits the receiving station to be several kilometers from the storage unit 13. Each channel or port of the storage unit 13 should have a capability to throttle the data flow into or out of the storage device 23 to provide for the wait times for resources to be free.

Another approach uses an implied switch by using a splitter/repeater in the fiber optic transmission path. This scheme connects several workstations 15 to a single fiber network using the splitter/repeater. Using this scheme requires that the data must include an attached address to identify the address of the corresponding workstation 15 to which the data is to be transmitted.

Yet another approach uses a network such as an fiber distribution data interface (FDDI) for the connection to each of the ports 16 of the storage device 23. Each such network provides a high speed data path with a capability of addressing the data to a selected destination on the network . Up to 28 such data paths may be connected to the storage device 23. This approach however yields a limited block transfer and lowers the overall communication capacity of each channel / port due to overhead message traffic necessary to arbitrate communication over the fiber channel.

High speed buffers 21 in the storage device 23 are adapted to capture an optimum block of data. The buffers 21 hold one service block in a pipelined data flow. Used at the highest speed these existing buffers 21 provide the service block buffering and the high speed data flow required of this architecture 20.

Buffers 21 are provided at each workstation 15 to receive data from the storage unit 13. These buffers 21 are the same size as those in the storage unit 13. Some storage units 13 have a throughput capability approaching 100 Mbits per second for uncompressed data or 200 Mbits per second for compressed data. Workstations 15 that are collocated with the storage unit 13 may not require buffer 21 and may be connected directly to the storage unit 13.

Data compression is generally performed at the workstations 15 in order that the data flow to and from the storage unit 13 be as small as possible. In addition, the use of data compression means that the storage requirement is reduced.

In summary, the present architecture permits one storage unit 13 to support a very large number of sources 11 and workstations 14. In a larger sense, the present invention supports a large number of image sources 11, processors 12, and workstations 15 for the imaging system 20. For other types of data or for mixed data types similar expansions of the resources of the system 20 without expanding the storage unit 13 are possible. Of course, the storage unit 13 must also have the appropriate amount of storage, as well as the rates and connections required required for the particular application.

The primary elements of the architecture 20 are the switch 22 attached to each input/output port 16 on the storage unit 13 and the associated buffers 21. The buffers 21 permit very rapid movement of the data into and out of the input/output port 16, freeing up the input/output port 16 as quickly as possible for use by another device 11, 12, 14, 15. In an implementation of the storage unit 13 that has been reduced to practice, the average waiting times are less than 30 milliseconds before the storage unit 13 becomes available.

The use of the buffers 21 permits the system 20 to "throttle" the data to or from the storage device 23 as required and to wait for the storage device 23 to become available. This throttling of the data flow permits the storage device 23 to achieve average data transfer rates of 30% to 50% of systems that does not employ this aspect of the present invention. Without the throttling capability, an extended transfer to a high rate device 11, 12, 14, 15 makes it very difficult for any other transfer to occur. Any other transfer may interrupt the data flow if the request for transfer appears at the wrong time, causing a short break in the data flow. For a tape unit or other similar device, the interruption can play havoc with the recording or playback of data.

Data that comes in blocks smaller than one service block may be grouped to fill one service block before transfer. Failure to group the data severely limits the throughput from the storage unit 13.

Compression may be used to reduce the number of service blocks required for large images or to increase the number of smaller images per service block. Movement of data through one input/output port 16 at 100 MBits per second can result in effective data rates after the compression has been removed that is greater than 200 Mbits per second. The high speed buffer 21 helps generate or absorb this high data rate. The compression along with the high speed of the compressed data flow makes the utilization of the input/output port 16 a small percentage of its throughput capacity and permits time sharing of the input/output port 16 with other elements through the use of the switch 22.

The addition of switches 22 and high speed buffers 21 permits the storage unit 13 to support nine times as many sources of data and eighteen times as many workstations 15 in a typical installation, compared to a storage unit 13 with direct connections. The number of devices 11, 12, 14, 15 expands from 28 for a typical installation to 120 or more. Full expansion on every input/output port 16 provides total connections to more than 300 devices.

Thus there has been described a new and improved image storage and dissemination architecture that provides for collection, storage, and distribution of images and other large data blocks to a large number of users. It is to be understood that the above-described embodiment is merely illustrative of some of the many specific embodiments which represent applications of the principles of the present invention. Clearly, numerous and other arrangements can be readily devised by those skilled in the art without departing from the scope of the invention.

## Claims

1. A data storage and dissemination architecture (20) that provides for collection, storage, and distribution of large data blocks to a large number of users, said architecture (20) characterized by:
a plurality of data sources (11) each comprising an output buffer;
a plurality of workstations (15) each comprising an input buffer and an output buffer;
a plurality of archive (14) each comprising an input buffer and an output buffer;
a plurality of processors (12);
storage unit means (13) having a plurality of input/output ports that each comprise an input buffer, an output buffer and a switch, and wherein a selected input/output port is selectively coupled to a plurality of the sources (11), to a plurality of workstations (15), to a plurality of processors (12), or to a selected plurality of archives (14), said storage unit means (13) further comprising a backplane bus (24) coupled to the input buffer and output buffer of each of the plurality of input/output ports and a plurality of storage devices coupled to the backplane bus (24) for storing data derived from the data sources (11), workstations (15), processors (12) and archives (14); and
a plurality of high speed fiber optic links (26) respectively coupled between the plurality of data sources (11) and a selected switch of the storage unit means (13), between the input and output buffers of the plurality of workstations (15) and a selected switch of the storage unit means (13), between the plurality of processors (12) and a selected switch of the storage unit means (13), and between the archive (14) and a selected buffer of the storage unit means (13).

2. An image storage and dissemination architecture (20) that provides for collection, storage, and distribution of images and other large data blocks to a large number of users, said architecture (20) characterized by:
a plurality of image sources (11) each comprising an output buffer;
a plurality of image workstations (15) each comprising an input buffer and an output buffer;
a plurality of archives (14) each comprising an input buffer and an output buffer;
a plurality of processors (12);
storage unit means (13) having a plurality of input/output ports that each comprise an input buffer, an output buffer and a switch, and wherein a selected input/output port is coupled to a selected plurality of the image sources (11), to a selected plurality of image workstations (15), to a selected plurality of processors (12), or to a selected plurality of archives (14), said storage unit means (13) further comprising a backplane bus (24) coupled to the input buffer and output buffer of each of the plurality of input/output ports and a plurality of storage devices coupled to the backplane bus (24) for storing images derived from the image sources (11), workstations (15), processors (12) and archives (14); and
a plurality of high speed fiber optic links (260 respectively coupled between the plurality of image sources (11) and a selected switch of the storage unit means (13), between the input and output buffers of the plurality of workstations (15) and a selected switch of the storage unit means (13), between the plurality of processors (12) and a selected switch of the storage unit means (13), and between the archive (14) and a selected buffer of the storage unit means (13).
